Europäisches Patentamt

**European Patent Office**

Office européen des brevets

(11) Veröffentlichungsnummer: **0 138 857**
**B1**

(12) # EUROPÄISCHE PATENTSCHRIFT

(45) Veröffentlichungstag der Patentschrift:
**14.01.87**

(51) Int. Cl.⁴: **C 02 F 1/78, A 61 L 2/20**

(21) Anmeldenummer: **84900963.4**

(22) Anmeldetag: **14.03.84**

(86) Internationale Anmeldenummer:
**PCT/CH 84/00039**

(87) Internationale Veröffentlichungsnummer:
**WO 84/03693 (27.09.84 Gazette 84/23)**

(54) **VERFAHREN UND VORRICHTUNG ZUR OZONISIERUNG EINES FLUIDUMS.**

(30) Priorität: **19.03.83 CH 1487/83**

(43) Veröffentlichungstag der Anmeldung:
**02.05.85 Patentblatt 85/18**

(45) Bekanntmachung des Hinweises auf die Patenterteilung:
**14.01.87 Patentblatt 87/3**

(84) Benannte Vertragsstaaten:
**AT BE CH DE FR GB LI LU NL SE**

(56) Entgegenhaltungen:
**CH-A-288 176**
**DE-A-2 347 826**
**DE-A-2 500 932**
**FR-A-1 159 012**
**FR-A-1 591 119**
**US-A-3 685 656**
**US-A-3 780 163**
**US-A-4 178 239**

(73) Patentinhaber: **HILTEBRAND, Peter, Blumenau 7, CH- 8184 Bachenbüllach (CH)**

(72) Erfinder: **HILTEBRAND, Peter, Blumenau 7, CH-8184 Bachenbülach (CH)**
Erfinder: **RUF, Arthur, Dr., Im Zimikerriet 8, CH-8603 Schwerzenbach (CH)**
Erfinder: **LAEDERACH, Herbert E., Dr., Kranichweg 30, CH- 3074 Muri (CH)**
Erfinder: **GSCHWEND, Karl, Dr., En Abbaye, CH-1891 Vérossaz (CH)**

(74) Vertreter: **Feldmann, Clarence Paul, c/o Patentanwaltsbüro FELDMANN AG Postfach Kanalstrasse 17, CH- 8152 Glattbrugg (CH)**

## Beschreibung

Ozon ist als eines der wirksamsten Oxidationsmittel bekannt und wird daher sowohl zur Entkeimung von Fluiden, z.B. Trinkwasser, Abwasser, Schlamm oder Abgasen, wie auch zur nichtmikrobiellen, d.h. chemischen Aufbereitung von Fluiden, z.B. Industrieabwässer mit organischen Belastungsstoffen, eingesetzt. Ein grosser Vorteil bei der Verwendung von Ozon zur Durchfürung entsprechender Verfahren ist dessen Eigenzerfall, d.h. überschüssiges Ozon zerfällt in Sauerstoff und verhindert damit eine weitere Umweltbelastung wie beispielsweise im Falle der Chlorentkeimung. Zudem entfällt jegliche Vorratslagerung, da Ozon an Ort und Stelle aus Sauerstoff, z.B. aus der Luft, hergestellt werden kann.

Der Einsatz von Ozon zur Durchfürung entsprechender Verfahren sowie Vorrichtung dazu sind seit langem bekannt. Ihrer verbreiteten Anwendung stand aber bisher ein zuverlässiger Betrieb des Ozonators entgegen.

Eine Analyse hat ergeben, dass für die Mehrzahl der Schadensfälle ein Rückströmen von mit dem ozonhaltigen Gas zu kontaktierendem Fluid in den Ozonator verantwortlich ist: Handelt es sich beim Fluid um eine Flüssigkeit, wird im Ozonator ein Kurzschlusstrom zwischen dessen Elektroden bewirkt. Handelt es sich um ein gasförmiges Fluid, kann entweder ein Auskondensieren von Feuchtigkeit, dass heisst Wasserdampf, an den gekühlten Elektrodenoberflächen eine Aenderung des Elektrodenabstandes bewirken, so dass lokale, hochenergetische Funkenentladung den Ozonator zerstören kann. Identisch läuft die Zerstörung des Ozonators durch an der Elektrodenoberfläche abgelagerte Feststoffpartikel ab.

Aus der US-Patentschrift 3 685 656 ist bekannt, zwischen Ozonator und Mischeinrichtung ein Rückschlagventil einzubauen, das ein Eindringen von Wasser aus dem Mischer in den Ozonator verhindert. Bei einer weiteren Anlage gemäss der deutschen Offenlegungsschrift 25 00 932 verhindert ein spezielles Zweiwegventil, das jedoch nur auf besonderen externen Befehl hin schliesst ein Rückströmen in den Ozonator.

Diese Massnahmen können jedoch nicht verhindern, dass aus dem dem Ozonator zugeführten sauerstoffhaltigen Gas, insbesondere Luft, Feuchtigkeit im Ozonator auskondensiert, obwohl heute meist spezielle Lufttrockner zur Aufbereitung der zu ozonisierenden Luft eingesetzt werden (DE 25 00 932). Derartige, meist auf der Basis von Silikagel als Trocknungsmittel arbeitende Lufttrockner können jedoch im Verlauf des Betriebes ihre Aufnahmekapazität überschreiten oder Betriebsstörungen im Regenerationsteil der Trockner können die Funktionssicherheit beeinträchtigen.

Eine weitere Beeinträchtigung der Betriebssicherheit des Ozonators kann dann eintreten, wenn der Gadurchsatz durch den Ozonator unter einen Minimalwert absinkt.

Zur Behebung dieses Nachteils wird in der deutschen Offenlegungsschrift 25 00 932 eine Anlage mit optischen Kontrollelementen für den Gasdurchsatz (in Form eines Rotameters) und der Feuchte (in Form eines Schauglases) vorgeschlagen. Diese Kontrollelemente weisen jedoch den Nachteil auf, dass sie beim Auftreten einer Störung den Ozonator nicht selbstständig abschalten können. Der Ozonator bedarf daher einer Ueberwachung durch Kontrollpersonal. Da entsprechende Störungen unerwartet und in den meisten Fällen rasch auftreten können, ist ein sicherer Betrieb des Ozonators nicht garantiert.

Es ist daher die Aufgabe der Erfindung, ein Verfahren und eine Vorrichtung anzugeben, das die geschilderten Nachteile nicht aufweist.

Die Lösung dieser Aufgabe wird durch die Merkmale des kennzeichnenden Teils des Anspruchs 1 bzw. 5 erreicht.

Erfindungsgemäss wird der Betriebstrom des Ozonators bei Auftreten eines zu hohen Feuchtegehaltes des zu ozonisierenden Gases, vorzugsweise Luft unterbrochen. Je nach Wahl des Feuchtesollwertes kann daher die durch den Wasserdampfgehalt des Gases beeinflusste Stickoxydbildung (Nebenreaktion der Ozonbildung) kontrolliert werden und dadurch die Ozonausbeute gesteigert werden. Die ständige Ueberwachung des Gasdurchsatzes gewährleistet zudem eine kontinuierliche und dem jeweiligen Bedarf angepassten Beschickung des Ozonators mit Frischgas und somit eine zuverlässige Ozonisierung des Fluids. Sinkt der Gasdurchsats unter den eingestellten Sollwert, Wird automatisch der Ozonator abgestellt und die Ozonisierung des Fluids, z.B. durch Abschaltung der Fluidpumpe unterbrochen. Die Fehlermeldesignale der Feuchtemesseinrichtung und der Gasdurchsatzmessvorrichtung werden zweckmässig mittels einer "oder-Verknüpfung" korreliert. Das daraus resultierende Signal kann z.B. zur Ansteuerung einer Alarmvorrichtung (akkustisch oder optisch) bzw. weiterer Elemente wie Fluidpumpe oder Wasserdampfentfernungsvorrichtung (z.B. zur Umsteuerung einzelnen Trocknersäulen) verwendet werden.

Die vollständige Entkeimung eines Fluids mit Ozon ist nur dann gewährleistet, wenn alle vermehrungsfähige Mikroorganismen abgetötet werden. Es wurde nun festgestellt, dass z.B. in Sporenform im Fluid vorliegende Mikroorganismen nicht allein durch die Einwirkung von Ozon abgetötet werden können. Es ist daher eine weitere Aufgabe der Erfindung, die Betriebssicherheit der Ozonisierung derart zu erhöhen, dass auch sporenhaltige Fluide vollständig entkeimt werden können. Diese Aufgabe wird durch die Merkmale der Ansprüche 2 bzw. 6 gelöst.

Bei der hier beschriebenen Verfahrensdurchführungsform wird das Fluid vor dem Ozonkontakt mit sauerstoffhaltigem Gas ozonfrei vorbelüftet. Diese Vorbelüftung dient zur Aktivierung der in Sporenform vorliegenden Mikroorganismen, deren Atmungssystem dadurch zu arbeiten beginnt. Erst bei aktiviertem Atmungssystem kann das anschliessend zugeführte Ozon seine Keimtötende Wirkung ausüben.

Damit die vom Ozon abgetöteten Mikroorganismen nicht als neuer Nährboden für allenfalls noch keimfähige Mikroorganismen dienen können, wird bei einer weiteren Ausgestaltung des Verfahrens das Fluid nach der Kontaktierung mit dem ozonhaltigen Gas filtriert. Das filtrierte Fluid wird anschliessend einem weiteren Mischer zugeführt, in dem eine zusätzliche Zudosierung von Ozon erfolgt.

Durch den Einsatz eines weiteren Mischers gemäss Patentanspruch 3 kann jedoch die Zwischenfiltration durchgeführt werden.

Da das aus dem Filter ausströmende Fluid weitgehend frei von aktiven, teilungsfähigen Mikroorganismen oder totem Zellmaterial ist, dient das im weiteren Mischer zudosierte Ozon grösstenteils zum Aufbau eines Ozonpuffers im Fluid. Damit kann verhindert werden, dass das vorzugsweise in einem Sammelbehälter gespeicherte ozonbehandelte Fluid einem neuen Mikroorganismenbefall ausgesetzt ist bzw. dass allenfalls den Filter in noch aktivem Zustand passierenden Mikroorganismen sich zu vermehren beginnen.

Damit bei einer notwendigen Reinigung des Filters dieses nicht mit zusätzlichen Mikroorganismen beladen wird, ist es zweckmässig, das Filter mit bereits ozonisiertem Fluid rückzuspühlen.

Es kann auch zweckmässig sein, eine Ausführungsform der Vorrichtung mit einer netzunabhängigen Stromversorgung auszurüsten. Eine derartige Vorrichtung kann z.B. als mobile Trinkwasseraufbereitungsanlage in abgelegenen Gebieten oder im Katastrophenfall eingesetzt werden.

Da der Mischer am Ozonator angeordnet ist, resultiert ein kürzestmöglicher Strömungsweg, dass heisst mit andern Worten eine kürzestmögliche Tansportzeit für das instabile Ozon von seiner Erzeugungsstelle bis in das Fluid. Ozonverluste durch Eigenzerfall fallen daher auf dem Transportweg nicht ins Gewicht.

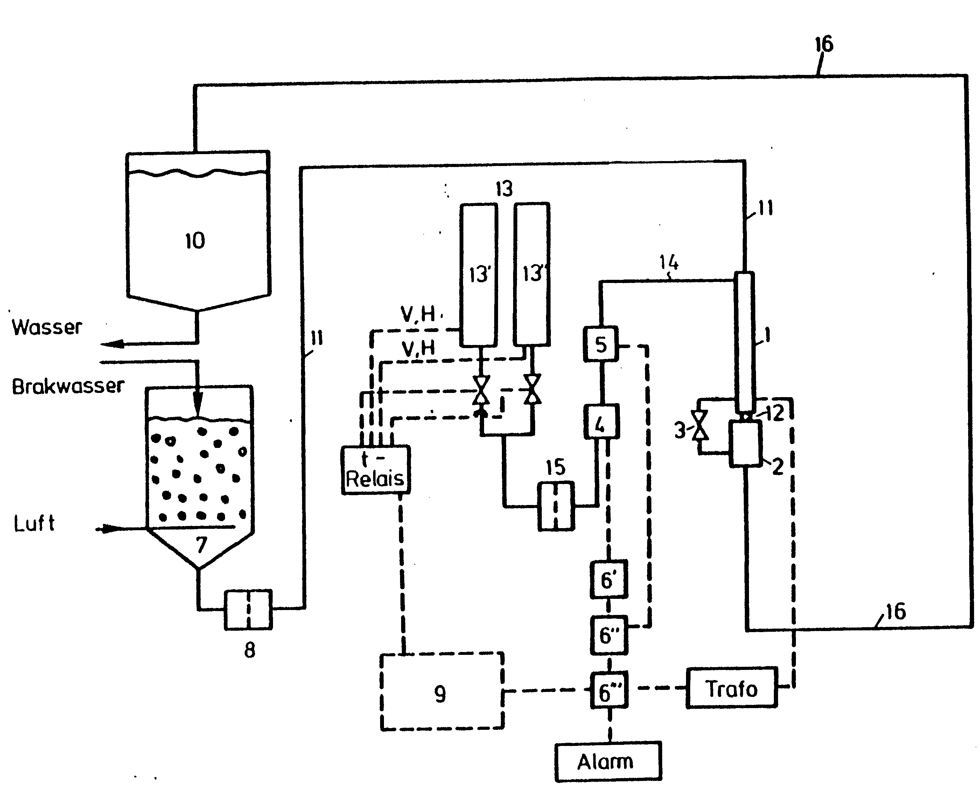

Im folgenden wird die Erfindung anhand eines Anwendungsbeispiels erläutert. Die einzige Figur zeigt das Verfahrensschema einer zur Aufbereitung von güllenverschmutztem Brakwasser verwendeten Vorrichtung.

Ein röhrenförmiger Ozonator 1 ist vertikal in eine Wasserleitung 11 eingebaut. Somit kühlt das Wasser den Ozonator. Unmittelbar an den Ozonator ist über ein PVC-Kupplungsstück 12 ein Venturimischer 2 angeflanscht. Das den Venturimischer durchströmende Wasser fördert aufgrund des sich innerhalb des Mischers aufbauenden Unterdrucks die zu ozonisierende Luft durch den silikagelgefüllten Trockner 13 und den Ozonator 1, in welchem ein Teil des Luftsauerstoffs zu Ozon gemäss bekanntem Reaktionsgleichgewicht umgeformt wird, und über die, das ohne Fremdenergiebedarf arbeitende Membranrückschlagventil 3, enthaltende Leitung in den Mischer 2 gelangt.

Im hochturbulenten Kontaktbereich des Mischers wird das ozonhaltige Gas feinblasig in das Wasser eindispergiert. Dadurch wird eine grosse spezifische Gas-Flüssigkeits-Kontaktfläche erzeugt und somit ein optimaler Ozonübergang in das Wasser erreicht.

In der Verbindungsleitung 14 zwischen Trockner 13 und Ozonator 1 sind je ein Luftdurchsatzmesser 5 und ein Messgerät 4 zur Bestimmung der relativen Luftfeuchte eingebaut. Diese dienen, wie im folgenden erläutert, der Ueberwachung der Betriebsbedingungen im Ozonator.

Umgebungsluft wird aufgrund des im Venturimischer erzeugten Unterdrucks in eine 13' der zwei Säulen 13', 13" des Trockners eingesogen. Das in der Trocknersäule vorhandene Silikagel entzieht der Luft den Wasserdampfgehalt. Zur Kontrolle dieses Trocknungsprozesses misst der Feuchtemesser 4 ständig die relative Feuchte der aus dem Trockner ausströmenden Luft. Steigt diese über einen von Hand einstellbaren Sollwert an, gibt die dem Feuchtemesser nachgeschaltete elektronische Vergleichsenheit 6' ein Signal aus. Die Grössenordung dieses Sollwerts ergibt sich je nach Anwendung und wird insbesondere durch die Temperatur der kältesten Stelle im Ozonator beeinflusst. Dabei ist entscheidend, dass an dieser Stelle der Taupunkt der zu ozonisierenden Luft nicht erreicht bzw. unterschritten wird. Da der Ozonator wassergekühlt ist, d.h. durch das zu entkeimende Brakwasser, kann näherungsweise dessen Temperatur zur Bestimmung das Sollwertes für die Vergleichseinheit 6' eingesetzt werden.

Es ist auch möglich, das Ausganssignal der Vergleichseinheit 6' zur Umsteuerung des Luftweges über die beiden Säulen 13', 13" zu verwerten, wobei gleichzeitig ein thermischer Regenerationsprozess in der bisher im Einsatz stehenden Säule 13' gestartet werden kann.

Da jedoch nebst dem Feuchtemesser 4 noch ein Luftdurchsatz messer 5 zur Kontrolle des Luftdurchsatzes durch den Ozonator dient, wird das genannte Signal in einer "oder-Verknüpfungseinheit"6" mit dem Signal des Feuchtemessers logisch verknüpft. Die Umsteuerung der Trocknersäulen geschieht allein über ein Zeitrelais. Der Luftdurchsatzmesser ist als Rotameter ausgebildet. Eine Lichtschranke oder ein Näherungsschalter taster die Position des Schwimmerkörpers ab. Sinkt dieser unter einen einstellbaren Sollwert, gibt der Luftdurchsatzmesser ein Signal aus. Der Sollwert des Luftdurchsatzmessers bestimmt sich dabei aus dem Bedarf an Ozon, der umgerechnet einem bestimmten Luftdurchsatz entspricht.

Das Ausgangssignal des "oder-Gliedes" 6" steuert somit sowohl bei Ueberschreiten des relativen Luftfeuchtesollwertes wie auch bei Unterschreiten des Luftdurchsatzsollwerts ein Relais 6''' an, das den Ozonatorstrom unterbricht und gleichzeitig eine Alarmeinheit (Horn, Sirene, oder Blinklicht) in Betrieb setzt. Da sich bei längerandauerndem Betrieb der Trockner Silikagelstaub bilden kann oder aus der Umgebung Staub durch den Trockner in die Verbindungsleitung 14 zum Ozonator eindringen kann, ist in Luftströmungsrichtung vor den Messeinrichtungen ein Feinstaubfilter 15 eingebaut.

Das den Mischer 2 verlassende Gemisch aus Wasser und ozonheltigem Gas wird über eine Rohrleitung 16, daren Länge sich aus der gewünschten Kontaktzeit der beioen Phasen ergibt, zu einem Sammelbehälter 10 geführt. In dieser Rohrleitung können zusätzlich statische Mischelemente eingebaut sein. Es ist aber auch möglich, vor dem Sammelbehälter ein Phasentrennelement, z.B. einen Gasabscheider, einzubauen. Letzteres wird insbesondere bei Badewasserentkeimungsanlagen bevorzugt, damit sich über dem Schwimmbad, das in diesem Fall die Funktion des Sammelbehälters übernimmt, keine die Gesundheit der Badenden gefährdende gasförmige Ozonschicht bilden kann.

Da insbesondere in Abwässern oft sporulierte, d.h. nicht atmungsaktive Mikroorganismen vorliegen, diese in der genannten Form jedoch durch das Ozon nicht vernichtet werden können, ist in einer Weiterbildung der Erfindung dem Ozonator eine Belüftungseinreichung 7, beispielsweise in Form eines Belüftungstanks oder einer Blasensäule, vorgeschaltet. Bedingt durch den damit eingetragenen Sauerstoff werden die sporulierten Organismen aktiviert und somit durch die Nachfolgende Ozonkontaktierung vernichtbar.

Ein oder mehrere Filter, z.B. Aktivkohlefilter oder Sandfilter, vor oder nach dem Mischer reinigen das Fluid von Feststoffen bzw. absorbieren dessen Restozongehalt. Diese Filter werden zur Reinigung vorzugsweise mit bereits ozonisiertem Fluid keimfrei rückgespühlt.

Da das mit Ozon zu behandelnde Fluid auch gasförmig, z.B. Abluft sein kann, wird anstelle des Venturimischers vorzugsweise ein Gasmischelement, z.B. ein statischer Mischer oder auch nur ein T-Stück eingebaut. Da derartige Elemente meist nicht selbstsaugend sind, muss zusätzlich ein Gasförderorgan für den Transport des im Ozonator zu ozonisierende Gas vorgesehen werden.

Sind grosse Mengen an Fluid zu entkeimen oder zu oxidieren, kann der Fluidstrom auf mehrere parallel arbeitende Ozonatoren aufgeteilt werden. Die vom Trockner aufbereitete Luft kann dabei über je einen einzigen Feuchte- bzw. Gasdurchsatszmesser oder pro Ozonator je einen dieser Messer geleitet werden. Entscheidend ist jedoch, dass zwischen jeden Ozonator und Mischer ein Rückschlagventil vorgesehen ist und die Mischer jeweils unmittelbar an den zugehörigen Ozonator angeflanscht sind.

Es ist aber auch möglich, einen Mehrfachmischer mit einzelnen angeflanschten Ozonatoren zu verwenden.

**Patentansrüche:**

1. Verfahren zur Entkeimung und/oder nichtmikrobiellen Aufbereitung eines Fluids mit ozonhaltigem Gas, wobei das aus einem Ozonator ausströmende Gas über ein ohne Fremdenergiebedarf arbeitendes, nur eine einzige Durchströmungsrichtung zulassendes Ventil (3) einem am Ozonator angeordneten Mischer, in dem das ozonhaltige Gas mit dem Fluid kontaktiert wird, zugeführt wird, wobei das Gas vor dessen Eintritt in den Ozonator durch mindestens eine Messvorrichtung zur Ueberwachung der relativen Feuchte und des Gasdurchsatzes geleitet wird, dadurch gekennzeichnet, dass die Messvorrichtung (4) bei Ueberschreiten eines einstellbaren Sollwertes für die relative Feuchte und bei Unterschreiten eines einstellbaren Sollwertes für den Gasdurchsatz den Betriebsstrom des Ozonators unterbricht und mindestens ein Alarmsignal und/oder ein Steuersignal ausgibt.

2. Verfahren nach Anspruch 1, dadurch gekennzeichnet, dass das Fluid vor dessen Eintritt in den Mischer mit Luft oder sauerstoffangereicherter Luft oder Sauerstoff kontaktiert Wird.

3. Verfahren nach Anspruch 1 oder 2, dadurch gekennzeichnet, dass das Fluid nach der Kontaktierung mit dem ozonhaltigen Gas filtriert und anschliessend einem weiteren Mischer, in dem wiederum ozonhaltiges Gas mit dem Fluid kontaktiert wird, zugeführt wird.

4. Verfahren nach Anspruch 1 bis 3, dadurch gekennzeichnet, dass der Sollwert für die relative Feuchte in Abhängigkeit der Temperatur an der kältesten Stelle im Ozonator so eingestellt wird, dass an dieser Stelle der Taupunkt der ozonisierenden Luft nicht unterschritten wird.

5. Vorrichtung zur Durchführung des Verfahrens nach einem der Ansprüche 1 bis 4 mit mindestens einem Ozonator (1) und einem am Ozonator angeordneten Mischer (2), wobei in der den Ozonator mit dem Mischer verbindenden Gasleitung mindestens ein ohne Fremdenergiebedarf arbeitendes, nur eine einzige Durchströmungsrichtung zulassendes Ventil (3) angeordnet ist und dass sie mindestens eine Messvorrichtung zur Ueberwachung der relativen Feuchte (4) des im Ozonator zu ozonisierenden Gases und des Gasdurchsatzes (5) aufweist, durch welche Messvorrichtung das zu ozonisierende Gas vor dessen Eintritt in den Ozonator geleitet wird, dadurch gekennzeichnet, dass die Messvorrichtung (4,5) Mittel aufweist, die bei Ueberschreiten eines einstellbaren Sollwertes für die relative Feuchte und bei Unterschreiten eines einstellbaren Sollwertes für den Gasdurchsatz den Betriebsstrom des Ozonators unterbrechen und mindestens ein Alarmsignal und/oder ein Steuersignal ausgeben.

6. Vorrichtung nach Anspruch 5, dadurch gekennzeichnet, dass sie eine Vorbelüftungseinrichtung (7) aufweist, in der das Fluid vor der Kontaktierung mit dem ozonhaltigen Gas mit Sauerstoff oder einem sauerstoffhaltigen Gas, z.B. mit Luft, kontaktiert wird.

7. Vorrichtung nach Anspruch 6, dadurch gekennzeichnet, dass der Vorbelüftungseinrichtung (7) ein Filter zur Entfernung der im Fluid enthaltenen Feststoffpartikel nachgeschaltet ist.

## Revendications

1. Procédé pour la stérilisation et/ou la préparation non microbienne d'un fluide avec du gaz contenant de l'ozone, dans lequel le gaz s'écoulant d'un ozoniseur est amené, en passant par une valve fonctionnant sans besoin d'energie extérieure, ne permettant qu'un seul sens de passage, à un mélangeur disposé sur l'ozoniseur, dans lequel le gaz contenant de l'ozone est mis en contact avec le fluide, le gaz étant conduit, avant son entrée dans l'ozoniseur, à travers au moins un dispositif de mesure pour surveiller l'humidité relative et le débit de gaz, caractérisé en ce que le dispositif de mesure, lorsque l'humidité relative devient supérieure à une valeur de consigne réglable et lorsque le débit de gaz devient inférieur à une valeur de consigne réglable, interrompt le courant de fonctionnement de l'ozoniseur et émet au moins un signal d'alarme et/ou un signal de commande.

2. Procédé selon la revendication 1, caractérisé en ce que le fluide, avant son entrée dans le mélangeur, est mis en contact avec de l'air, ou de l'air enrichi en oxygène, ou de l'oxygène.

3. Procédé selon l'une des revendications 1 et 2, caractérisé en ce que le fluide, après la mise en contact avec le gaz contenant de l'ozone, est filtré et ensuite amené à un autre mélangeur dans lequel, à nouveau, du gaz contenant de l'ozone est mis en contact avec le fluide.

4. Procédé selon l'une des revendications 1 à 3, caractérisé en ce que l'on règle la valeur de consigne de l'humidité relative sous la dépendance de la température à l'endroit le plus froid de l'ozoniseur, de façon telle qu'en cet endroit l'humidité relative ne devienne pas inférieure au point de rosée de l'air ozonisant.

5. Appareil pour la mise en oeuvre du procédé selon l'une des revendications 1 à 4, muni d'au moins un ozoniseur (1) et d'un mélangeur (2) disposé sur l'ozoniseur, dans lequel, dans le tuyau de gaz reliant l'ozoniseur au mélangeur est disposée au moins une valve (3) fonctionnant sans besoin d'énergie extérieure, ne permettant qu'un seul sens de passage, l'appareil présentant au moins un dispositif de mesure pour surveiller l'humidité relative (4) du gaz à ozoniser dans l'ozoniseur et le débit (5) de gaz, dispositif de mesure à travers lequel le gaz à ozoniser est conduit avant son entrée dans l'ozoniseur, caractérisé en ce que le dispositif de mesure (4, 5) présente des moyens qui, lorsque l'humidité relative devient supérieure à une valeur de consigne réglable et lorsque le débit de gaz devient inférieur à une valeur de consigne réglable, interrompent le courant de foncitonnement de l'ozoniseur et émettent au moins un signal d'alarme et/ou un signal de commande.

6. Appareil pour la mise en oeuvre du procédé selon la revendication 5, caractérisé en ce qu'il présente un dispositif d'aération préalable (7) dans lequel le fluide, avant la mise en contact avec le gaz contenant de l'ozone, est mis en contact avec de l'oxygène ou un gaz contenant de l'oxygène, par exemple avec de l'air.

7. Appareil pour la mise en oeuvre du procédé selon la revendication 6, caractérisé en ce qu'à la suite du dispositif d'aération prélable (7) est branché un filtre pour l'élimination des particules de solide contenues dans le fluide.

## Claims

1. Process for the sterilization and/or non-microbial preparation of a fluid with ozone-containing gas, in which the gas flowing out of the ozonizer is supplied via a valve operating without any external energy requirement and only permitting a single flow direction to a mixer arranged on the ozonizer and in which the ozone-containing gas is contacted with the fluid and prior to the gas entering the ozonizer it is passed through at least one measuring device for monitoring the relative humidity and the gas flow rate, characterized in that on exceeding an adjustable set value for the relative humidity and on dropping below an adjustable set value for the gas flow rate, the measuring device interrupts the working current of the ozonizer and supplies at least one alarm signal and/or a control signal.

2. Process according to claim 1, characterized in that prior to the fluid entering the mixture, it is contacted with air or oxygen-enriched air or oxygen.

3. Process according to claims 1 or 2, characterized in that, following contacting with the ozone-containing air, the fluid is filtered and then supplied to a further mixer, in which once again ozone-containing gas is contacted with the fluid.

4. Process acoording to claims 1 to 3, characterized in that the set value for the relative humidity is so adjusted as a function of the temperature at the coldest point in the ozonizer, that at this point there is no drop below the dew point of the ozonizing air.

5. Apparatus for performing the process according to one of the claims 1 to 4, with a least one ozonizer (1) and an mixer (2) arranged on said ozonizer, the gas line connecting the ozonizer to the mixer containing at least one valve (3) operating without an external energy requirement and only permitting a single flow direction and has at least one measuring device for monitoring the relative humidity (4) of the gas to be ozonized in the ozonizer and the gas flow rate (5), the gas to be ozonized being passed through the measuring device before it enters the ozonizer, characterized in that the measuring device (4, 5) has means which interrupt the working current of the ozonizer on exceeding an adjustable set value for the relative humidity and on dropping below an adjustable set value for the gas flow rate and supply at least one alarm signal and/or a control signal.

6. Apparatus according to claim 5, characterized in that it has a preaeration device (7), in which the fluid is contacted with oxygen or an oxygen-containing gas, e.g. air prior to contacting with the ozone-containing gas.

7. Apparatus according to claim 6, characterized in that the preaeration device (7) is followed by a filter for removing the solids particles contained in the fluid.